# NEUE EUROPÄISCHE PATENTSCHRIFT

(11) **EP 1 128 789 B2**
(45) Veröffentlichungstag und Bekanntmachung des Hinweises auf die Entscheidung über den Einspruch: **05.12.2007**
(45) Hinweis auf die Patenterteilung: 25.06.2003
(21) Anmeldenummer: 00976072.9
(22) Anmeldetag: 24.11.2000
(51) Int. Cl.: A61F 2/66

(54) **FUSSPROTHESE**
FOOT PROSTHESIS
PROTHESE DU PIED

(30) Priorität: 24.12.1999 DE 19962851
(43) Veröffentlichungstag der Anmeldung: 05.09.2001
(73) Patentinhaber: BIEDERMANN MOTECH GmbH, 78054 Villingen-Schwenningen (DE)
(72) Erfinder: BIEDERMANN, Lutz, 78048 VS-Villingen (DE); SCHNEIDER, Urs, 72074 Tübingen (DE)
(74) Vertreter: Hofer, Dorothea
(86) Internationale Anmeldenummer: PCT/EP2000/011717
(87) Internationale Veröffentlichungsnummer: WO 2001/047444

(56) Entgegenhaltungen:
- DE-U- 29 707 416
- DE-U- 29 820 904
- DE-U- 29 823 435
- DE-U- 29 920 434
- US-A- 4 547 913
- US-A- 5 116 384
- US-A- 5 800 570

## Beschreibung

Die Erfindung betrifft eine Fußprothese. Aus dem Prospekt BIEDERMANN MOTECH, Unterschenkel-Systeme, Seite 15 ist eine Fußprothese bekannt, die aus einem ersten Federelement und einem damit verbundenen zweiten Federelement gebildet ist. Das erste Federelement erstreckt sich von dem Zehenbereich bis zum Unterschenkelbereich und ist von oben her gesehen konvex ausgebildet. Im Unterschenkelbereich trägt das freie Ende einen Adapter zum Verbinden mit dem Unterschenkelelement einer Prothese. Etwa im Bereich der Fußballen ist das Federelement auf seiner Unterseite mittels Schrauben fest mit der Oberseite des vorderfußseitigen Endes eines zweiten Federelementes verbunden, welches sich mit seinem freien Ende zum Fersenbereich hin erstreckt. Das erste Federelement ist aus einem Karbonfaserverbundmaterial gebildet und hat das Aussehen einer Blattfeder mit einer Breite von etwa 4 cm im Ballenbereich und einer Dicke von 3 bis 4 mm.

Die US 4,547,913 zeigt ebenfalls eine aus zwei Blattfedern bestehende Fußprothese. Ein erstes Federelement erstreckt sich vom Zehenbereich zum Unterschenkelbereich, ein zweites vom Fersenbereich zum Unterschenkelbereich. Im Unterschenkelbereich sind beide Federelemente miteinander verbunden. Je nach Ausführungsform ist entweder das erste oder das zweite Federelement über den Unterschenkelbereich hinaus verlängert als Beinabschnitt der Prothese. Aus der US 5,800,570 ist eine Fußprothese nach dem Oberbegriff des Anspruchs 1 bekannt.

Aufgabe der Erfindung ist es, eine Fußprothese mit noch besseren Gangeigenschaften zu schaffen.

Diese Aufgabe wird durch eine Fußprothese nach Patentanspruch 1 gelöst.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht einer ersten Ausführungsform der Erfindung;
- Fig. 2: eine entsprechende Seitenansicht einer zweiten Ausführungsform;

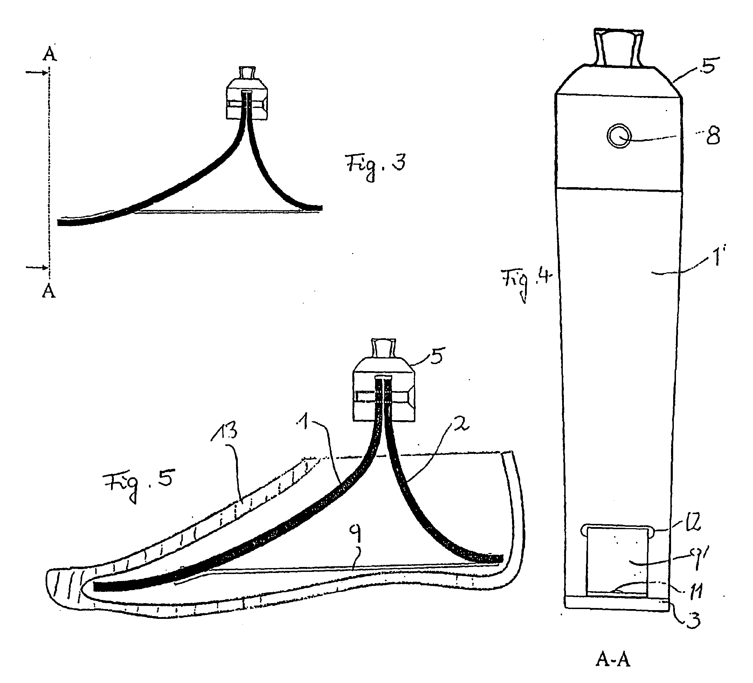

- Fig. 3: eine Darstellung entsprechend der in Fig. 2;
- Fig. 4: eine Draufsicht auf den in Fig. 3 gezeigten Gegenstand in Richtung der Pfeile A'-A; und
- Fig. 5: den in Fig. 1 gezeigten Gegenstand in einem geschnitten dargestellten Kunstfuß.

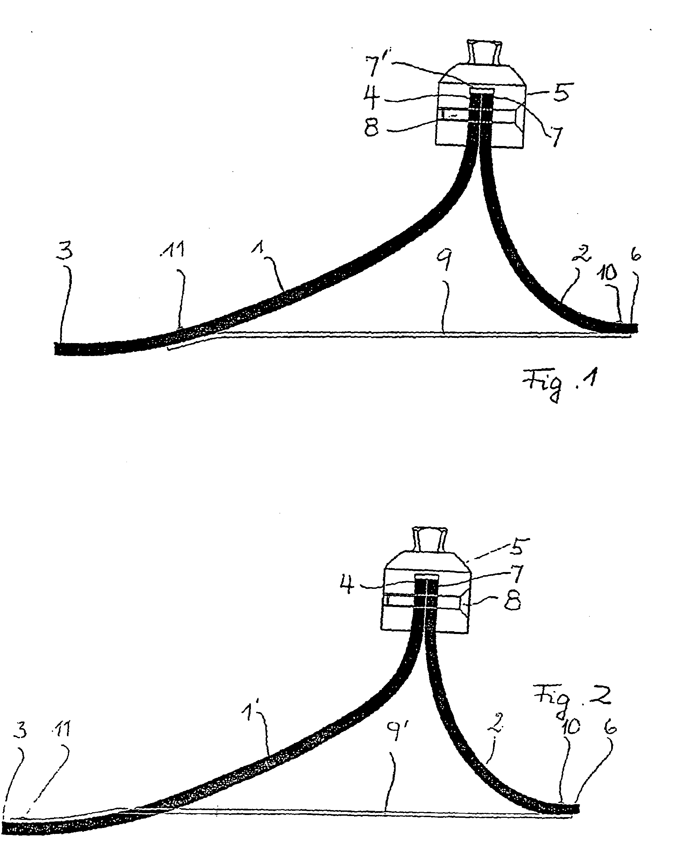

Die in Fig. 1 gezeigte erste Ausführungsform weist ein erstes Federelement 1 und ein zweites Federelement 2 auf. Die beiden Federelemente sind jeweils von oben her gesehen konkav ausgebildet. Das erste Federelement 1 erstreckt sich mit einem freien Ende 3 von dem Zehenbereich bis zu seinem gegenüberliegenden freien Ende 4, welches in einem mit einem Unterschenkelteil einer Prothese verbindbaren Adapter 5 gehalten ist. Das zweite Federelement 2 erstreckt sich mit seinem einen freien Ende 6 von der Ferse bis zu seinem anderen freien Ende 7, welches in dem Adapter 5 gehalten ist.

Die Biegung der beiden Federelemente 1, 2 ist so gewählt, daß die beiden bodenseitigen freien Enden 3, 6 in Ruhestellung auf dem Boden flach aufliegen und das die gegenüberliegenden freien Enden 4, 7 mit ihrer konvexseitigen Oberfläche parallel zueinander aneinander stoßen, wie dies in der Figur gezeigt ist, und so in einem Schlitz 7' des Adapters 5 gehalten und mit einer Arretierschraube 8 in dem Adapter 5 befestigt werden.

Wie aus den Figuren ersichtlich ist, sind der Vorderfußbereich des ersten Federelementes 1 und der Fersenbereich des zweiten Federelementes 2 mittels eines Zugelementes 9 miteinander verbunden. Wie am besten aus Fig. 4 ersichtlich ist, ist das Zugelement bandartig ausgebildet und ist als ein reckfreies Zugband ausgebildet. Das Zugelement 9 ist mit seinem einen Ende mittels Nieten 10 möglichst nah am freien Ende 6 des zweiten Federelementes 2 zugfest verbunden und mit seinem anderen freien Ende mittels Nieten 11 nahe beim freien Ende des ersten Federelementes 1 mit diesem verbunden. Dabei ist die Verbindungsstelle der Nieten 11 so gewählt, daß das erste Federelement 1 in der Normalstellung noch einen Abstand vom Boden aufweist. Das hat den Vorteil, daß während des Laufens das Zugelement 9 nicht stets auf dem Boden aufsitzt und so geschont wird. Die Verbindungsstelle mit den Nieten 11 soll so nah wie möglich an dem freien Ende 3 des ersten Federelementes liegen.

Die so gebildete Fußprothese weist ein besonders vorteilhaftes Gangverhalten vom Auftreten des Fußes im Fersenbereich bis zum Abrollen über den Vorderfuß auf.

Die in Fig. 2 gezeigte Ausführungsform stimmt bezüglich der Form der Federelemente, der Verbindung mit dem Adapter und dem Vorsehen eines Zugelementes überein.

Damit der Vorteil erreicht wird, daß das Zugelement 9' so nah wie möglich an den freien Enden 3, 6 mittels Nieten 10, 11 befestigt wird und somit das Zugelement an den äußersten freien Enden angreift, andererseits aber auch der Vorteil erreicht wird, daß das Zugelement nicht vollständig auf dem Boden aufliegt, ist in einem Abstand von dem freien Ende 3, in dem das erste Federelement 1 gerade einen ausreichenden Abstand vom Boden aufweist, ein am besten in Fig. 4 sich quer zur Längsrichtung des ersten Federelementes 1' erstreckender Schlitz 12 vorgesehen. Dieser dient dazu, daß Zugelement 9' durch den Schlitz 12 hindurch zum vorderen freien Ende 3 des ersten Federelementes 1 zu führen. Von dem Schlitz 12 aus liegt das Zugelement 9', wie am besten aus Fig. 2 ersichtlich ist, eng an der konvexen des Federelementes 1' an und ist mit diesem mittels Nieten 11 im Zehenbereich fest verbunden.

Alternativ ist es natürlich auch möglich, das Zugelement 9 auf der Unterseite des ersten Federelementes 1 bis zum freien Ende zu führen, wenn in Kauf genommen wird, daß das Zugelement dann auf dem Boden aufliegt.

In Fig. 5 ist die zuerst beschriebene Ausführungsform in einem Kunstfuß 13 in an sich bekannter Weise eingesetzt.

## Patentansprüche

1. Fußprothese mit einem sich von dem Zehenbereich zum Unterschenkelbereich hin erstreckenden ersten Federelement (1), einem sich von dem Fersenbereich zu dem Unterschenkelbereich erstreckenden zweiten Federelement (2), wobei die Federelemente (1, 2) mit ihren unterschenkelseitigen ersten Enden (4, 7) miteinander verbunden sind, wobei
ein Zugelement (9, 9') **vorgesehen ist,** welches mit seinem einen Ende mit dem Vorderfußbereich (3) und mit seinem anderen Ende mit dem Fersenbereich (6) der Federelemente (1, 2) verbunden ist, **dadurch gekennzeichnet, daß** die beiden Federelemente (1, 2) als Blattfedern ausgebildet sind und an ihrer Verbindungsstelle mit ihren bodenseitigen Oberflächen aneinanderliegen.

2. Fußprothese nach Anspruch 1, **dadurch gekennzeichnet, daß** die verbundenen Enden (6, 7) mit einem Adapter (5) zum Verbinden mit einem Unterschenkelteil verbunden sind.

3. Fußprothese nach Anspruch 1, **dadurch gekennzeichnet, daß** das Zugelement (9, 9') bandförmig ausgebildet ist.

4. Fußprothese nach einem der Ansprüche 1 bis 3, **dadurch gekennzeichnet, daß** das Zugelement (9, 9') mit seinen Enden jeweils bodenseitig mit den Federelementen (1, 2) fest verbunden ist.

5. Fußprothese nach einem der Ansprüche 1 bis 3, **dadurch gekennzeichnet, daß** das Zugelement (9, 9') am vorderen Ende des ersten Federelementes (1) auf dessen Oberseite befestigt ist und dadurch eine in einem Abstand vom vorderen Ende vorgesehenen schlitzförmige Ausnehmung (12) in dem Federelement zum zweiten Federelement (2) hin geführt ist.

6. Fußprothese nach einem der Ansprüche 1 bis 5, **dadurch gekennzeichnet, daß** die Federelemente (1, 2) von oben her gesehen konvex ausgebildet sind.

7. Fußprothese nach einem der Ansprüche 1 bis 6, **dadurch gekennzeichnet, daß** die Federelemente (1, 2) aus Karbonfaserverbundmaterial und das Zugelement (9, 9') aus einem reckfreien Material gebildet sind.

## Claims

1. Foot prosthesis comprising a first spring member (1) extending from the toe region to the lower leg region, and a second spring member (2) extending from the heel region to the lower leg region, wherein the first ends (4, 7) of the spring members (1, 2) adjacent to the lower leg region are connected to one another, wherein a tension member (9, 9') is provided which is connected at one end with the forefoot region (3) and at the other end to the heel region (6) of the spring members (1, 2), **characterised in that** the two spring members (1, 2) are formed as leaf springs and their undersides rest against one another at their connecting point.

2. Foot prosthesis according to claim 1, **characterised in that** the connected ends (6, 7) are connected to an adapter (5) for connection to a lower leg part.

3. Foot prosthesis according to claim 1, **characterised in that** the tension member (9, 9') is in the form of a strip.

4. Foot prosthesis according to any one of claims 1 to 3, **characterised in that** the tension member (9, 9') is fixedly connected at each of its ends to the underside of the spring members (1, 2).

5. Foot prosthesis according to any one of claims 1 to 3, **characterised in that** the tension member (9, 9') is fixed to the upper surface of the forward end of the first spring member (1) and from there is guided through a slot-type opening (12) provided in the spring member at a distance from the forward end, towards the second spring member (2).

6. Foot prosthesis according to any one of claims 1 to 5, **characterised in that** the spring members (1, 2) are convex when viewed from above.

7. Foot prosthesis according to any one of claims 1 to 6, **characterised in that** the spring members (1, 2) are formed from carbon-fibre composite material and the tension member (9, 9') is formed from a non-stretch material.

## Revendications

1. Prothèse de pied avec un premier élément formant ressort (1) qui s'étend de la région des orteils à la région de la jambe, un second élément formant ressort (2) qui s'étend de la région du talon à la région de la jambe, les deux éléments formant ressort (1, 2) étant reliés entre eux par leurs premières extrémités (4, 7) du côté de la jambe dans laquelle un élément de traction (9, 9') est prévu , qui est relié à une extrémité avec la région de l'avant-pied (3) et par l'autre extrémité à la région de talon (6) des éléments formant ressort (1, 2), **caractérisée en ce que** les deux éléments formant ressort (1, 2) sont conformés comme des ressorts à lames et reposent l'un sur l'autre par leurs surfaces orientées vers le sol à leur point d'assemblage.

2. Prothèse de pied selon la revendication 1 **caractérisée en ce que** les extrémités assemblées (6, 7) sont reliées à un adaptateur (5) servant à la liaison avec une partie jambière.

3. Prothèse de pied selon la revendication 1, **caractérisée en ce que** l'élément de traction (9, 9') est en forme de bande.

4. Prothèse de pied selon l'une ou l'ensemble des revendications 1 à 3 **caractérisée en ce que** l'élément de traction (9, 9') est fixé aux éléments formant ressort (1, 2) à ses extrémités du côté du sol

5. Prothèse de pied selon l'une ou l'ensemble des revendications 1 à 3, **caractérisée en ce que** l'élément de traction (9, 9') est fixé à l'extrémité antérieure du premier élément formant ressort (1) sur la face supérieure de celui-ci et est guidé vers le second élément formant ressort (2) à travers une ouverture (12) en forme de fente prévue dans l'élément formant ressort à distance de la première extrémité.

6. Prothèse de pied selon l'une ou l'ensemble des revendications 1 à 5 **caractérisée en ce que** les éléments formant ressort (1, 2) sont convexes en vue de dessus.

7. Prothèse de pied selon l'une ou l'ensemble des revendications 1 à 6, **caractérisée en ce que** les éléments formant ressort (1, 2) sont composés de matériau composite en fibres de carbone et l'élément de traction (9, 9') est fait d'un matériau sans allongement.
